# DEMANDE DE BREVET EUROPEEN

(11) **EP 2 366 791 A1**
(43) Date de publication de la demande: **21.09.2011**
(21) Numéro de dépôt: 10305259.3
(22) Date de dépôt: 16.03.2010
(51) Int. Cl.: C12N 15/82, C07K 14/415, C12N 15/11, A01H 5/00

(54) **Méthode pour modifier la date de floraison d'une plante**

(71) Demandeur: Biogemma, 75001 Paris (FR); Genoplante-Valor, 75015 Paris (FR)
(72) Inventeur: La désignation de l'inventeur n'a pas encore été déposée
(74) Mandataire: Flesselles, Bruno F.G.

(57) **Abrégé**

La présente invention se rapporte au domaine de l'amélioration des plantes, en particulier de la modification de leur date de floraison, par manipulation du gène Prec31.

SEQUENCE LISTING

<110>  BIOGEMMA
       GENOPLANTE-VALOR

<120>  Méthode pour modifier la date de floraison d'une plante

<130>  BRV 25 - EP PRIO

<160>  8

<170>  PatentIn version 3.3

<210>  1
<211>  365
<212>  PRT
<213>  Zea mays

<400>  1

Met Gly Thr Gly Ala Val Ala Val Ala Ala Pro Asp Arg Ala Lys Gln
1               5                   10                  15

Arg Arg Ala Gly Gly His Leu Trp Lys Lys Ala Leu Leu His Phe Ser
            20                  25                  30

Leu Cys Phe Val Met Gly Phe Phe Thr Gly Phe Ala Pro Ser Ser Ser
        35                  40                  45

Ser Ser Trp Lys Ser Ala Ala Ala Thr Pro Gln His His Pro Pro His
    50                  55                  60

Arg Pro Gly Asp Arg Leu Ala Ala Ser Arg Val Ala Val Asp Ala Arg
65                  70                  75                  80

Ala Thr Leu Ser Pro Pro Ala Glu Ala Ala Asp Ala Leu Ser Ala Gly
                85                  90                  95

Ala Thr Val Asp Leu Gly Asp Asp Glu Glu Asp Ala Ala Gln Arg Arg
            100                 105                 110

Leu Leu Ile Val Val Thr Thr Thr Arg Ser Gly Pro Gly Glu Arg Arg
        115                 120                 125

Arg Arg Arg Gly Glu Leu Leu Arg Leu Ala His Thr Leu Arg Leu Val
    130                 135                 140

Arg Pro Pro Val Val Trp Val Val Val Glu Pro Ala Ala Asp Ala Pro
145                 150                 155                 160

Ala Thr Ala Glu Val Leu Arg Gly Thr Gly Val Met Tyr Arg His Leu

                165                 170                 175

Ala Phe Lys Pro Glu Asp Asn Phe Thr Thr Ala Asp Ala Glu Ala His
            180                 185                 190

Val Gln Arg Asn Ala Ala Leu Ala His Val Glu Lys His Arg Leu Ala
        195                 200                 205

Gly Val Leu His Phe Ala Asp Ala Ala Gly Val Tyr Asp Val Gly Phe
    210                 215                 220

Phe Glu Gln Ile Arg Gln Ile Glu Ala Phe Gly Thr Trp Pro Val Ala
225                 230                 235                 240

Thr Met Ser Ala Gly Glu Lys Lys Val Val Val Glu Gly Pro Leu Cys
                245                 250                 255

Ser Ala Ser Lys Val Val Gly Trp Phe Ser Arg Asp Phe Asn Asp Gly
            260                 265                 270

Thr Thr Arg Ser Val Thr Tyr Asn Thr Glu Ala Asp Leu Asn Pro Ala
        275                 280                 285

Gly Ala Ala Gly Thr Arg Ala His Thr Ile Asp Val Ser Gly Phe Ala
    290                 295                 300

Phe Asn Ser Ser Ile Leu Trp Asp Pro Glu Arg Trp Gly Arg Pro Thr
305                 310                 315                 320

Ser Leu Pro Asp Thr Ser Gln Asp Ser Ile Lys Phe Val Gln Glu Val
                325                 330                 335

Leu Leu Glu Asp Arg Ala Lys Leu Lys Gly Ile Pro Ser Asp Cys Ser
            340                 345                 350

Gln Ile Met Val Trp Gln Tyr Ser Val Pro Ser Ser Arg
        355                 360                 365

<210> 2
<211> 6760
<212> DNA
<213> Zea mays

<400> 2
tctcaaccgg tcgtaccttt tatatagggg agatctggac atgtttctag acgagatcca 60

acagactcac acggataggc taggataatc acgcatgaga taaatacttg tatccgagtt 120

aacctaatca cgaaccgttc ggactaccct gcggaccgtt caggccacaa ggtgaaacga 180
aatcgtacga ccgtgcctgt gtgccaaata tgatgctcaa caactgaaag catgattttt 240
tttaaaaaaa actgaagttt atttttaaaa cttaaaatcc atctcggtaa aaatgaaatt 300
ggcttgaaat ctcattttcg gtgacctcca aaaattaaaa aacgaaaact ttaaccctga 360
aatgaaccca cgcaataccg gatcctattg tgctggaacc ggacaaaagg aaaactgtac 420
tttataccgg actgcatgct aatgctaatc tattaaccgc actccactcc accccctccc 480
gccctgtcca caccaaccac gctcggaggc tcccctttca ttttcacctc gctccggggc 540
caagtcgcca gttcaccacc ttcacttcac ttcgcaccgg accaccggcc gtattcattc 600
ctcctccctc cctcctcgcc atacggctgc agcatcttcc cctcaccctc ctcattaccc 660
cgcgtctctc cctcaaaact actcattgct ctcaccgttc cattgccccc gcggccccgc 720
cgtacgctca ttcccggcta gctcctcctc cgcacggcca tgatcttgag ttcttggcat 780
tgatgacgtt ctagtaccga gcgagccagc ccctctttaa cccaccttaa caataaacac 840
agcctgcccc caccggccac ctcttcttcc cccgtgcagc tcgagctgat tcggcgggct 900
ctgccccaac atccacccac cttcgtccaa tcccgatcca agaccgtgcc gtgatccggc 960
agcagcttcc gcgcggccat gggcacgggc gcggtggcgg tggcggcgcc ggaccgggcc 1020
aagcagcggc gggccggcgg ccacctgtgg aagaaggcac tgctccactt ctccctctgc 1080
ttcgtcatgg gcttcttcac gggcttcgcg ccttcctcct cctcctcctg gaagtccgcc 1140
gccgccaccc cccagcacca cccgccgcac cgtcccgggg accgcctcgc cgcctcccgc 1200
gtggccgtcg acgcgcgcgc caccctgtcg ccgcccgcgg aggccgccga tgcgctctcc 1260
gccggcgcca cggtggactt gggcgacgac gaggaggacg ccgcccagcg ccgcctcctg 1320
atcgtggtca ccaccacgcg ctccggcccc ggggagcgcc gccgccgccg gggcgagctg 1380
ctgcgcctcg cccacacgct gcgcctcgtg cgcccgcccg tcgtctgggt cgtcgtcgag 1440
cccgccgccg acgcgcccgc caccgctgag gtgctccgcg gcaccggcgt catgtacagg 1500
cacctcgcct tcaagcccga ggacaacttc accaccgccg acgccgaggc tcacgtgcag 1560
cggaacgccg cgctcgcgca tgtcgagaag caccgactcg ccggcgtcct gcacttcgcc 1620
gacgccgccg gcgtctacga cgtcggcttc ttcgaacaga tccgccagat cgagtcagta 1680
ccccctcttc ccctcccacc accttcaaat ctcgccttct tccttctatt tcatgccatg 1740
attgttttta attaatgaaa tgactcatgc tttcattgat ccttcatctt cttacttatt 1800
aaaatccgtc taggctatta agatgcaaat tatttccaag caagtttcct gatttctttc 1860
ctatttttgg tttccaagct caaagattgg agctttgcct ggcccctctt tcaatttcac 1920
atctaactga tctccatttc cagcacatgc tgttcttcca gaatgtatgt tgcttgaatc 1980

cgaatcacaa tgcctaaaat tgcaattcca gctcataaat tccggttctt tttttttccc 2040

caccgactag gactacgact agggactcct cataactaat catcgtttct taattcagta 2100

ccataagtta ttttatttac tcgtctaaat aaatgcttta ttagccgtgt agtttggtac 2160

ataaataaat aattaaaaac ataagctatc attagcgggg gaataagggt gggacaatgt 2220

gtgtctcgca tgtacttacc aaactattct tcttaatgct ctgaaggggg aaaaaaggga 2280

ggaggcactg tagattctca gagaacttca actattctct ccatttctct gatgcgagga 2340

gtcaattatt ggatctagct tatcactttc ctgagtccct ttcgccccca ccaaacaatg 2400

cttcttttaa tctttcgctt ttgctcaccc ccaaggcccc aacggacaaa tgtgttcatg 2460

ccaaccaccc caactagttt aatttagact ttaaacacgt atcatgagca aagccacaat 2520

tattttatgt ggtagtggta gcactgctgt tagtcactga caaatgggcc tgtaagagtg 2580

tcggtggcac ctaacagatt ggaaagtcag tgagaacatg acatccttga attgactcct 2640

gagatgttgt tactcctata ctggggaagc aacatgctgg ggggaaatag ctacagccta 2700

caaaagatag caggcaggga gaaaaatagt agtaaccgtt atgccatggc cattgaaact 2760

gattgaacta aagtggagag aaggtcctag ttctatccac accattaatt tcttcctcca 2820

accccataat gtccatctga tatacaccgc agaaagttgc ccacgtattc ggtgcagttt 2880

tcctatactg gtgcacacag caccacctgt gatctagatc ttctggagcc cgcaagaaac 2940

tccaaaaaga acttcgtttg caaaatggca gtatgaatct tcatttctgt gcagaccacc 3000

agttgtcttt ggagattttt ctcatgagga gtttggaaag tcttgcaaaa atgagatttc 3060

acatttcatt ttaccaacat ttgatgttga ttattgggtt tcttacttgc atgaccatga 3120

attctcatca gagtatgaaa tccagtattt ccaccagagc atgacactag tatagcatct 3180

gaagtttgtt ctgaccgccc ctttaacccc tttgcttatc catctgaaca ttgttagcgg 3240

atgctgttac aagtttagat gcttatagat ataaagcaat ctacatttca aaaaaaaaaa 3300

tacatgcata aagcaatctt actcagcatc aaattccgaa accttttgcc tcttaacttg 3360

aaaacacatc aacatttgac tcaccttttg cattcacttt tcccgatggt agcgttcttt 3420

cattgatggg tgcttgcttt cataaagaga ggggatcaga ccttggttta gtacaatggt 3480

actagtagat cactactaca gtagtgctca tgccacgtgg tagcacagtg gcgatcttcc 3540

ctggaagaac agtctttttc gcttagagga acatgatggg gtaaaaacag cctcttttcc 3600

tcaagcaaga ttttacctgc acctgggcac caaaacatct gtgcttgttt ttctagtttg 3660

ccttctcttt tttgtgtgtg tgtccctttt aatctttaca gtcctcgaga tgcgtgcaca 3720

tctgcctttg tggcccctga tgctttttgt ggggctccag cataataatg caacagaggt 3780

aggagcatca attatgcctt gtcgcacgta ggtcatagca tgcctgtcct agaactacca 3840

gccaccagaa acaaatgagt gcaggtgaac actacagtga ctataagtgt ggtttactat 3900

actaattggc tgggttttga ccgcagttaa tgtttcttgc gtctggttga gatttagcat 3960

tggattcttt taatcctagg aggctgaaat ttggttacgg tttaccttaa tgtttgaata 4020

agctcgtttg ttccagttga gttttactat acctatgtag ttgtgtttga ctgcatattg 4080

ggaacacgtc tgcttactca agtttccatt gatcgtgaac cgttgcatca tcccgtagtt 4140

tgtttgatct ggatttgtca agattattca aactctggaa ttggtagtgg aacaaattag 4200

gcatctgcat gtgcagccgt gcaggcaagt ggagggtgta gctgtactgt atagtttaca 4260

tgcaataact cagtctattc cttgtatgaa atgatttgga tgagcggtca aaactatagt 4320

agtcagcatg ttaaagatgt gtcaatggag gtatcccctc ctatcacatg ttctttcgag 4380

ttgaggaaaa tcagatgcgt ctgttttttt cttccttcga attaaaatca attggctgat 4440

gtgtggttag tggtgccatt ctagtcaagg tttggtggca caatgctttt caattccata 4500

atccataatt cttctgaccc agtcaagatg agacacatag atcacaggca aagctacctc 4560

ttgcgtcaca catgttttgg tgcatgttgc atggttcttg ttgaatttca tctcagatgt 4620

tggtgccttt agatgttttt ttttctaaca tttcaaaatg cgcagggcgt ttggtacatg 4680

gcctgtggca acaatgtcgg cgggcgagaa gaaagtggta gtggagggtc cactgtgcag 4740

tgcctcaaag gtcgtcggtt ggttctcaag ggacttcaac gacggaccca cccgttcagt 4800

gacgtataac acggaagctg acctgaatcc tgctggtgcc gctggtacac gagcccacac 4860

gatcgacgtc tcaggttttg cgttcaatag ctctattttg tgggatcctg agcggtgggg 4920

acggccaacg tcattaccag acacttcaca ggttagtcca accccgcctt tgtggagaga 4980

gtgttagtga ccgagcaagt ctagttcaat taaattttgc tttggcatga tacaacatac 5040

tccctccgat tttttttatt tgacgtttgt tagtataaag ttgaactatc tcaaacaaaa 5100

aatgggggta gtacttagca tttagcaagc tatggtatgt ggttgactca gtgaagggac 5160

tggttctcag atattagcaa tctttggctg tccatctctt gagaatgtcg tcgtataaaa 5220

actctgaatt gagattatta atttgaagat gaaatgttaa gccattggca ttagtaattt 5280

gaagtgttat gtcaaggaat ggtttcatac tctgaattgt tttccttctg ttacttgaaa 5340

aatgaaaggc atcagtttct aatataacag tgtctacgat gtgggctgtg attggcttgt 5400

atgtgttaca aggtcctgta aaatcttcct ttttcttgat aaaaaacgtg ctaaacacaa 5460

tctcaaagaa aaaaaaaggc aagcggttgg aacagattgg atgctgacct tgtcaatggt 5520

tcaagtcaat cactgcaggt caagcatgtc cactcatgtt ccttctgaac aaggttcggg 5580

tatagtagat aaattgtctg gattcaattc atgatctttt tttatctaga ataatcactc 5640

tcaacatatc tcatgatcta cttgtcatgt gcaagcaata agctaccaac agttagttac 5700

tgatgtgatt gcaggactcg atcaagttcg tgcaagaagt gctacttgaa gaccgagcta 5760

agctgaaggg gatcccctcc gactgctccc agatcatggt gtggcagtac agcgtgccca 5820

gctcgcgata gagggcagcc gagaggacgc acaagaggcg tcgcaacctc gcaggcaggg 5880

gaagaactgc agggggttgg atcggagtcg ctgtaaatgt ggtgggcgat ggtaacggca 5940

aggaaagcaa gaaggctgat tactaagggt tctttttctc tctctctcct ttttgtgttc 6000

cctttttctt tcccctcagt tttagttttc aggtttggga ggggggccat gaatgatgaa 6060

ctgtccgtgg gagcaaaaga attgcgatag tatttattca gattcatttg tgattcttta 6120

gcctggtcgg ttggtcctgt tgtgtttggg ttaaaaaaaa tggcagtggc gtgaggaatg 6180

gagaaagttt ttattgtgtt atggcatgaa atcccagcaa ggcagcagca attaacatct 6240

ggagaacata aaaatcatgt gcaaagggaa agaaaaaaaa gagagaagag actgaatctg 6300

cgctcaggtc ttgctgtcat cgtccctggt gtttccttgc gggacagtgt ttatttgctt 6360

ttgaaaaagg tctgtgcttt gcttgtaagt agagatggca atgggtaccc gaaacccgat 6420

gggtaaaaac cctattaggg tacgggtatg gcgaattttg atacccatgg gtattttatt 6480

gggtcatttg ttatgcccat cgggtacggt gggcgtgagt atgttctctg ttgccccata 6540

cccgctaccc gatggggaac ccgctaacat atgacacgtg ggtcggattt atatgtccca 6600

tataaaatga atttgctctc gttatttgat aaccctaaga ccctaacgcg gccaccaggg 6660

caccacgcac caggcaccag gccagccgcc agacggacca tccagaagtc ttgtatattc 6720

aaatattaag tattgtgtat tgaacaatta gatttgaagg 6760

<210> 3
<211> 1095
<212> DNA
<213> Zea mays

<400> 3
atgggcacgg gcgcggtggc ggtggcggcg ccggaccggg ccaagcagcg gcgggccggc 60

ggccacctgt ggaagaaggc actgctccac ttctccctct gcttcgtcat gggcttcttc 120

acgggcttcg cgccttcctc ctcctcctcc tggaagtccg ccgccgccac cccccagcac 180

cacccgccgc accgtcccgg ggaccgcctc gccgcctccc gcgtggccgt cgacgcgcgc 240

gccaccctgt cgccgcccgc ggaggccgcc gatgcgctct ccgccggcgc cacggtggac 300

ttgggcgacg acgaggagga cgccgcccag cgccgcctcc tgatcgtggt caccaccacg 360

cgctccggcc ccggggagcg ccgccgccgc cggggcgagc tgctgcgcct cgcccacacg 420

ctgcgcctcg tgcgcccgcc cgtcgtctgg gtcgtcgtcg agcccgccgc cgacgcgccc 480

gccaccgctg aggtgctccg cggcaccggc gtcatgtaca ggcacctcgc cttcaagccc 540

gaggacaact tcaccaccgc cgacgccgag gctcacgtgc agcggaacgc cgcgctcgcg 600

catgtcgaga agcaccgact cgccggcgtc ctgcacttcg ccgacgccgc cggcgtctac 660

gacgtcggct tcttcgaaca gatccgccag atcgaggcgt ttggtacatg gcctgtggca 720

acaatgtcgg cgggcgagaa gaaagtggta gtggagggtc cactgtgcag tgcctcaaag 780

gtcgtcggtt ggttctcaag ggacttcaac gacgggacca cccgttcagt gacgtataac 840

acggaagctg acctgaatcc tgctggtgcc gctggtacac gagcccacac gatcgacgtc 900

tcaggttttg cgttcaatag ctctattttg tgggatcctg agcggtgggg acggccaacg 960

tcattaccag acacttcaca ggactcgatc aagttcgtgc aagaagtgct acttgaagac 1020

cgagctaagc tgaaggggat cccctccgac tgctcccaga tcatggtgtg gcagtacagc 1080

gtgcccagct cgcga 1095

<210> 4
<211> 19
<212> DNA
<213> Artificial

<220>
<223> amorce

<400> 4
tgagtggaca tgcttgacc 19

<210> 5
<211> 20
<212> DNA
<213> Artificial

<220>
<223> amorce

<400> 5
cgtataacac ggaagctgac 20

<210> 6
<211> 19
<212> DNA
<213> Artificial

<220>
<223> amorce

<400> 6
gattgcagga ctcgatcaa 19

<210> 7

<211>  20
<212>  DNA
<213>  Artificial

<220>
<223>  amorce

<400>  7
tttaacccaa acacaacagg                                                   20


<210>  8
<211>  25
<212>  DNA
<213>  Artificial

<220>
<223>  amorce

<400>  8
cttcgtccat aatggcaatt atctc                                             25

## Description

La présente invention se rapporte au domaine de l'amélioration des plantes, en particulier de la modification de leur date de floraison.

Le développement des plantes est composé d'une phase végétative et d'une phase de reproduction. La floraison représente la transition entre ces deux phases.

La floraison est ainsi un événement essentiel dans le cycle de vie des plantes et est d'une grande importance pour la sélection de plantes adaptées à leur environnement.

La date de floraison d'une plante reflète sa capacité d'adaptation à son environnement et notamment aux conditions climatiques, et aux stress externes.

Le maïs possède un organe mâle et un organe femelle, qui fleurissent de manière asynchrone.

Un nombre importants de signaux environnementaux (tels que la longueur du jour, la température (vernalisation), la disponibilité de nutriments, d'eau ou de phytohormones) contrôlent la transition entre les phases végétatives et reproductives, et sont donc susceptibles d'influencer la date de floraison.

La date de floraison du maïs est également influencée par différents facteurs génétiques. Ainsi, Buckler et al (Science, 2009, 325, p 714-718) décrivent l'identification de régions chromosomiques du maïs (QTL pour *Quantitative Trait* Loci) influençant la floraison de ces plantes. Ces régions ont été identifiées en utilisant une grande population de plantes. Les auteurs précisent que les différences observées entre les lignées consanguines ne sont pas causées par quelques gènes ayant un effet important, mais plutôt par l'effet cumulatif de plusieurs QTL. Ainsi, les QTL identifiés présentent plutôt des effets mineurs (au maximum 1,5 jours de précocification.

De fait, il existe au moins 80 gènes de floraison chez *Arabidopsis* (voir Blazquez et al 2001 (EMBO Rep. 2001 Dec;2(12):1078-82). Le nombre d'orthologues de ces gènes chez le maïs est très important : ainsi, Buckler *et al.,* parlent de 1000 gènes de floraison *d'Arabidopsis* ayant des homologues chez le maïs.

Différents documents ont décrit l'identification de gènes impliqués dans la floraison dans d'autres espèces. On peut notamment citer WO 2006/068432, qui décrit les gènes OsMADS50, OsMADS51, OsMADS56, OsMADS14 (tronqué), OsTRXI, OsVIN2, OsCOL4, et OsCOL8. Ces différents gènes montrent un effet sur le riz, plante autogame. Les inventeurs de la demande précédente ont ensuite étudié plus en détail le rôle de OsMADS51 (Kim et al, Plant Physiol. 2007 Dec;145(4):1484-94), confirmant les effets rapportés dans WO 2006/068432, et précisant une séquence (architecture) génétique de la floraison chez le riz.

Les inventeurs ont identifié un gène codant pour une protéine qui sera désignée en tant que Prec31 dans la présente demande, et dont la séquence d'acides aminés est représentée par SEQ ID N° 1.

Chez le maïs, un mutant dans le gène codant pour cette protéine présente une date de floraison mâle et femelle précoces par rapport à la plante quasi-isogénique non mutante.

La présente invention se rapporte ainsi à un procédé pour avancer la date de floraison d'une plante, caractérisé en ce que l'on inhibe totalement ou partiellement l'expression et/ou l'activité dans ladite plante, d'une protéine dénommée Prec31, dont la séquence polypeptidique possède au moins 75% d'identité avec la séquence SEQ ID N° 1.

Dans le cadre de la présente description, les éléments de comparaison (avance de la date de floraison, date de floraison plus précoce ou plus tardive) le sont par rapport à des plantes quasi-isogéniques, sauf en ce qui concerne l'objet de l'invention (inhibition de Prec31, présence d'une mutation ou d'un transgène destiné à inhiber Prec31). Une plante quasi isogénique possède un génome identique à au moins 95 % au génome de la plante objet de l'invention, sauf en ce qui concerne la présence des éléments inhibant Prec31. Une lignée quasi-isogénique peut être obtenue par rétrocroisement de la plante objet de l'invention avec la lignée avec laquelle on souhaite établir la comparaison.

Le principe en est rappelé ci-dessous : On réalise une série de rétrocroisements (back-cross) entre la lignée avec laquelle on souhaite effectuer une comparaison et la lignée portant le(s) élément(s) inhibant Prec31.

Au cours des rétrocroisements, on peut sélectionner les individus porteurs de(s) élément(s) inhibant Prec31, et ayant recombiné le plus petit fragment de la lignée donneuse autour de ce transgène. En effet, grâce aux marqueurs moléculaires, on sélectionne les individus ayant pour les marqueurs proches du gène, le génotype de la lignée élite.

De plus, il est également possible d'accélérer le retour vers le parent élite grâce aux marqueurs moléculaires répartis sur l'ensemble du génome. A chaque rétrocroisement, seront choisis les individus ayant le plus de fragments issus du parent élite récurrent.

Avec une bonne mise en oeuvre, dès la quatrième génération, on peut obtenir une lignée quasi isogénique de la lignée élite, c'est-à-dire identique à la lignée élite de départ, mais ayant intégré le(s) élément(s) inhibant Prec31. Le fragment intégré porte le(s) élément(s) inhibant Prec31, ainsi que d'éventuelles régions chromosomiques flanquantes issues du maïs de départ (notamment les lignées bien connues A188 ou Hi-II, utilisées pour la transformation, lorsque l'on évalue l'effet d'un transgène dans le maïs).

Ladite plante est notamment le maïs, le riz, le sorgho, le millet, le blé et autres céréales à paille, ou une plante fourragère.

On définit ici comme protéine Prec31, toute protéine dont la séquence polypeptidique présente au moins 75 %, de préférence au moins 85 %, ou au moins 90 %, avantageusement au moins 94 %, et de manière tout à fait préférée au moins 95 %, de manière plus préférée au moins 97 % d'identité avec la séquence SEQ ID N° 1 sur une fenêtre de comparaison la plus large possible, correspondant de préférence à la totalité de la séquence SEQ ID N° 1. Sauf précision contraire, les pourcentages d'identité indiqués ici sont établis à l'aide du logiciel BLAST2 (ALTSCHUL et al., Nucleic Acids Res.,25, 3389- 3402,1997) en utilisant les paramètres par défaut (matrice BLOSUM62 pour les comparaisons de protéines, avec des pénalités de 11 pour un « gap » ouvert, 1 pour un « gap » d'extension, 50 pour un « gap x_dropoff », une valeur d'attente aléatoire de 10, une taille de mots (word size) de 3, et pas de filtre).

L'inhibition totale ou partielle de l'expression et/ou de l'activité de la protéine Prec31 peut être obtenue de diverses manières, connues par l'homme du métier.

De façon préférée, on diminue l'expression du gène codant pour Prec31, par mutagenèse, ou bien par inhibition ou modification de sa transcription ou de sa traduction.

Le gène Prec31 est entendu comme comprenant les régions codantes et non codantes (régions 5' ou 3' non traduites, introns) du gène.

La mutagenèse du gène codant pour Prec31 peut intervenir au niveau de la séquence codante ou des séquences de régulation de l'expression, notamment du promoteur, ou de la région 3' non traduite (3'UTR). On peut par exemple procéder à la délétion de tout ou partie dudit gène et/ou à l'insertion d'une séquence exogène. A titre d'exemple, on citera la mutagenèse insertionnelle : on produit un grand nombre d'individus dérivant d'une plante active pour la transposition d'un élément transposable, (élément AC ou Mutator chez le maïs), et on sélectionne, par exemple par PCR, les plantes chez lesquelles une insertion s'est effectuée dans le gène de Prec31, ou à proximité avec conséquence sur la traduction ou l'expression.

On peut aussi utiliser toute autre méthode connue dans l'art pour inhiber le gène codant pour Prec31. Ainsi, on peut procéder à la mutation des gènes par insertion d'un élément transposable ou d'un ADN de transfert (T-DNA). On peut aussi effectuer une mutagenèse physique ou chimique, notamment par l'utilisation d'EMS, de rayons X ou d'ultraviolets (voir notamment McCallum et al, Plant Physiol.,123, 439-442, 2000). Les mutations intéressantes ont pour conséquence de décaler le cadre de lecture et/ou d'introduire un codon stop dans la séquence et/ou de modifier le niveau de la transcription et/ou de la traduction du gène et/ou de rendre l'enzyme moins active que la protéine sauvage.

Les plantes ainsi mutées sont criblées par exemple par PCR, en utilisant des amorces situées dans le gène cible. Toutefois, on peut aussi utiliser d'autres méthodes de criblage, comme les Southern Blots ou un criblage via la méthode AIMS décrite dans WO 99/27085 (pour détecter les insertions), en utilisant des sondes spécifiques des gènes cibles, ou les méthodes de détection de mutations ponctuelles ou de petites insertions/délétions utilisant des endonucléases particulières (Cel I, Endo I) telles que décrites dans WO 2006/010646.

Dans un autre mode de réalisation, l'inhibition est obtenue par transformation de la plante avec un vecteur contenant un construit sens ou antisens du gène cible. Ces deux méthodes sont connues pour permettre, dans certaines conditions, l'inhibition du gène cible. On utilise également la méthode de l'interférence d'ARN (RNAi) qui est particulièrement efficace pour l'extinction de gènes dans les plantes. Cette méthode est bien connue de l'homme du métier et consiste en la transformation de la plante avec un construit produisant, après transcription, un duplex double-brin d'ARN, dont l'un des brins est complémentaire à l'ARNm du gène cible (pour revue sur les techniques d'inhibition post-transcriptionnelles, Chicas et Macino, EMBO reports, 21(11), 992-996,2001 ; pour revue sur l'utilisation des ARN interférents, Hannon, Nature, 418,244-251, 2002).

La présente invention a ainsi notamment pour objet l'utilisation d'au moins un polynucléotide choisi parmi :
a) un polynucléotide codant pour une protéine Prec31 telle que définie ci-dessus ;
b) un polynucléotide complémentaire d'un polynucléotide a) ci-dessus ;
c) un fragment d'au moins 12 nucléotides consécutifs, d'un polynucléotide a) ou b) ci-dessus, ou capable de s'hybrider sélectivement avec ledit polynucléotide ;
d) un polynucléotide permettant la production d'un duplex utilisable pour l'interférence d'ARN (RNAi), ledit un polynucléotide contenant un polynucléotide X contenant au moins 12 nucléotides, de préférence au moins 15, avantageusement au moins 20, et de manière tout à fait préférée au moins 50 nucléotides capable de s'hybrider sélectivement avec un polynucléotide défini en a), ainsi qu'un polynucléotide Y complémentaire dudit polynucléotide X
pour obtenir une plante présentant une date de floraison plus précoce.

Un polynucléotide codant pour Prec31 est un polynucléotide contenant l'information génétique permettant la synthèse de la protéine Prec31 représentée par SEQ ID N° 1, ou d'une protéine présentant au moins 75 %, de préférence au moins 85 %, ou au moins 90 %, avantageusement au moins 94 %, et de manière tout à fait préférée au moins 95 %, de manière plus préférée au moins 97 % d'identité avec SEQ ID N° 1.

Ceci englobe notamment l'ADN génomique, dont la séquence SEQ ID N° 2 représentée en annexe représente un allèle, ainsi que l'ADNc correspondant (SEQ ID N° 3). La partie codante comprend les nucléotides 979-1674, 4667-4948 et 5712-5831 (codon STOP inclus) de SEQ ID N° 2

Un fragment spécifique d'un polynucléotide a) ou b) ci-dessus est un fragment dudit polynucléotide dont la séquence n'est pas trouvée dans d'autres gènes de la même plante.

Un polynucléotide capable de s'hybrider sélectivement avec un polynucléotide a) ou b) ci-dessus, est un polynucléotide qui lorsqu'il est hybridé en conditions stringentes avec une banque d'acide nucléique de la même plante (notamment une banque d'ADN génomique, ou d'ADNc) produit un signal d'hybridation détectable au moins 2 fois supérieur, et de préférence au moins 5 fois supérieur au bruit de fond avec ledit polynucléotide, mais ne produit aucun signal détectable avec d'autres séquences de ladite banque.

L'homme du métier est capable de définir la stringence des conditions d'hybridation, qui dépendent de la taille et de la composition en bases du polynucléotide concerné, ainsi que de la composition du mélange d'hybridation (notamment pH et force ionique). Généralement, des conditions stringentes, pour un polynucléotide de taille et de séquence données, sont obtenues en opérant à une température inférieure d'environ 5 °C à 10 °C à la température de fusion (Tm) de l'hybride formé, dans le même mélange réactionnel, par ce polynucléotide et son complémentaire.

La présente invention concerne en particulier pour objet un procédé pour avancer la date de floraison d'une plante, par inhibition totale ou partielle de la protéine Prec31 endogène de ladite plante, comprenant la transformation de ladite plante avec une construction d'ADN recombinant comprenant un polynucléotide tel que défini ci-dessus, placé en orientation sens ou en orientation antisens, ou pouvant être transcrit en ARN double brin, sous contrôle transcriptionnel d'un promoteur approprié.

L'invention se rapporte également à une méthode pour retarder la date de floraison d'une plante, par surexpression d'une protéine Prec31 dans ladite plante. Cette surexpression est réalisée par transformation de ladite plante avec une construction d'ADN recombinant comprenant un polynucléotide tel que défini ci-dessus sous contrôle transcriptionnel d'un promoteur approprié. Les plantes transgéniques ainsi obtenues sont également objet de l'invention.

La présente invention a également pour objet des constructions d'ADN recombinant, comprenant un polynucléotide tel que défini ci-dessus. Ces constructions sont notamment des cassettes d'expression, comprenant un polynucléotide tel que défini ci-dessus, sous contrôle transcriptionnel d'un promoteur approprié. Ces cassettes d'expression peuvent également contenir d'autres éléments de régulation, en particulier des éléments de régulation de la transcription tels que des terminateurs, amplificateurs.

Des vecteurs recombinants qui comprennent des polynucléotides tels que définis selon l'invention ou une cassette d'expression selon l'invention sont également objets de l'invention.

De tels vecteurs peuvent également comprendre d'autres éléments, par exemple un ou plusieurs marqueurs de sélection, et sont notamment utilisables pour l'obtention de plantes transgéniques.

A titre d'exemples non-limitatifs de promoteurs utilisables dans le cadre de la présente invention, on peut citer les promoteurs constitutifs, tels que le promoteur 35S du virus de la mosaïque du chou-fleur (CaMV), ou ses dérivés, le promoteur du virus de la mosaïque des nervures de manioc (CsVMV) (WO 97/48819), le promoteur de l'ubiquitine ou le promoteur Actine-Intron-actine, du riz (McElroy et,al., Mol. Gen. Genet.,231, 150-160,1991 ; GenBank S 44221).

Des promoteurs inductibles ou tissu-spécifiques peuvent également être utilisés. Ceci permet de n'induire l'inhibition de Prec31 qu'à certains stades du développement de la plante, dans certaines conditions environnementales, ou dans certains tissus cibles, comme par exemple, les tiges, les feuilles, les graines, les spathes, le cortex ou le xylème.

On utilise également avantageusement d'autres éléments de régulation de la transcription tels des terminateurs, et notamment le terminateur 3'NOS de la nopaline synthase (Depicker et al., J. Mol. Appl. Genet., 1, 561-573,1982), ou le terminateur 3'CaMV (Franck et al. Cell, 21,285-294,1980 ; GenBank V00141).

Les gènes marqueurs de sélection utilisables dans le cadre de la présente invention sont notamment des gènes conférant une résistance à un antibiotique tel que l'hygromycine, la kanamycine, la bléomycine ou la streptomycine, ou à un herbicide (EP 0 242 246) tel que le glufosinate, le glyphosate ou la bromoxynil. Le gène nptll qui confère la résistance à la kanamyine peut ainsi être utilisé.

La transformation des plantes peut s'effectuer par de nombreuses méthodes, connues en elles-mêmes de l'homme du métier.

On peut par exemple transformer des cellules végétales, des protoplastes ou des explants, et régénérer une plante entière à partir du matériel transformé. La transformation peut ainsi être réalisée, par transfert des vecteurs conformes à l'invention dans des protoplastes, notamment après incubation de ces derniers dans une solution de polyéthylèneglycol (PG) en présence de cations divalents (Ca2+) selon la méthode décrite dans l'article de Krens et al. (Nature, 296,72-74,1982) ou par électroporation notamment selon la méthode décrite dans l'article de Fromm et al. (Nature, 319,791-793, 1986). On peut également utiliser un canon à gène permettant la projection, à très grande vitesse, de particules métalliques recouvertes des séquences d'ADN d'intérêt, délivrant ainsi des gènes à l'intérieur du noyau cellulaire, notamment selon la technique décrite dans l'article de Finer et al. (Plant Cell Report, 11,323- 328,1992) ou la micro-injection cytoplasmique ou nucléaire.

On utilise préférentiellement la méthode de transformation par *Agrobacterium tumefaciens,* notamment selon la méthode décrite par Ishida et al. (1996, Nature Biotechnol. 14, 745-50).

La présente invention a également pour objet les cellules végétales et les plantes transgéniques ou mutantes susceptibles d'être obtenues par un procédé conforme à l'invention, et les cellules et plantes contenant un polynucléotide recombinant ou une cassette d'expression tels que définis ci-dessus. L'invention se rapporte également aux cellules végétales et aux plantes obtenues après mutation du gène codant de Prec31 de telle sorte qu'il en résulte une inhibition de l'expression et/ou l'activité de la protéine.

Bien entendu, la présente invention englobe les plantes dérivées de plantes selon l'invention, qui sont notamment les descendants, notamment les hybrides issus d'un croisement impliquant au moins une plante selon l'invention, obtenus par semis ou par multiplication végétative, des plantes selon l'invention ou obtenues directement ou indirectement par un procédé selon l'invention.

L'invention comprend également les cellules et tissus végétaux, ainsi que les organes ou parties de plantes, y compris feuilles, tiges, racines, fleurs, fruits, et/ou graines obtenues à partir d'une plante conforme à l'invention.

De façon préférée, l'invention s'applique au maïs et aux cellules et tissus issus de maïs.

L'invention se rapporte en particulier à un maïs ou un grain de maïs présentant un allèle du gène Prec31, appelé delta-Prec31 comprenant une insertion d'un transposon dans la région 3' non-traduite, 297 nucléotides après la codon STOP de la région traduite, ledit allèle étant présent dans un échantillon représentatif de graines déposées au NCIMB sous le numéro NCIMB 41706.

Des graines possédant l'allèle delta-Prec31 ont été déposées au NCIMB Limited, Ferguson Building, Craibstone Estate, Bucksburn, Aberdeen, Scotland, AB21 9YA, UK, le 15 mars 2010, selon les dispositions du Traité de Budapest, sous le numéro NCIMB 41706.

La présente invention a aussi pour objet un procédé de sélection de maïs, caractérisé en ce qu'il comprend la recherche d'un allèle du gène de Prec31 possédant une mutation résultant en une date de floraison anticipée dudit maïs, par rapport à un maïs ne possédant pas cet allèle.

Dans un cas préféré, ladite mutation résulte en une inhibition totale ou partielle de l'expression et/ou de l'activité de Prec31.

Ledit allèle peut être recherché par détection directe de la mutation responsable de l'inhibition; il peut également être recherché par détection de la forme allélique associée à cette mutation d'un polymorphisme en déséquilibre de liaison avec celle-ci.

Ces allèles mutants ainsi identifiés peuvent ensuite être introgressés dans des lignées choisies, et notamment dans des lignées élites à savoir des lignées présentant un potentiel agronomique et commercial important.

Dans un mode de réalisation particulier, on recherche un polymorphisme localisé au nucléotide 5089 de SEQ ID N° 2. Lorsque le nucléotide est un T, la floraison est avancée. Lorsque le polymorphisme est une délétion, la floraison est plus tardive.

Dans un mode de réalisation particulier, on recherche un polymorphisme localisé au nucléotide 5025 de SEQ ID N° 2. Lorsque le nucléotide est un G, la floraison est avancée. Lorsque le nucléotide est un T, la floraison est plus tardive.

Dans un mode de réalisation particulier, on recherche un polymorphisme localisé au nucléotide 4961 de SEQ ID N° 2. Lorsque le nucléotide est un A, la floraison est avancée. Lorsque le nucléotide est un C, la floraison est plus tardive.

Dans un mode de réalisation particulier, on recherche la présence de deux polymorphismes aux nucléotides 5089 et 5025 de SEQ ID N° 2. Dans un autre mode de réalisation, on recherche la présence de deux polymorphismes aux nucléotides 5089 et 4961 de SEQ ID N° 2. Dans un autre mode de réalisation, on recherche la présence de deux polymorphismes aux nucléotides 5025 et 4961 de SEQ ID N° 2.

Dans un autre mode de réalisation, on recherche la présence des trois polymorphismes aux nucléotides 4961, 5025 et 5089 de SEQ ID N° 2.

Il est à noter que l'on observe souvent la présence de deux haplotypes A /G / T, et C / T / -, correspondant à un allèle précoce pour le premier, et un allèle tardif pour le second.

De façon préférée, le maïs transgénique selon l'invention est un maïs « élite ». L'homme du métier connaît bien la définition d'un maïs élite. Il s'agit d'un maïs destiné à générer des hybrides destinés à être commercialisés par croisement avec un autre maïs élite. Un maïs élite est défini comme tel en relation avec le territoire envisagé pour la commercialisation, ainsi que le(s) caractère(s) agronomique(s) recherché(s) pour la descendance hybride. Il s'agit notamment d'un maïs pouvant être inscrit dans un catalogue de référence.

Ainsi, si la descendance est destinée à l'alimentation animale, on évaluera les caractères de rendement (recherche d'un rendement élevé et régulier en tonnage de matière sèche à l'hectare), d'ingestibilité et de digestibilité, lorsque l'on évalue la nature « élite du maïs ». Si la descendance est destinée à la production de biocarburants, tels les biofuels ou les biogaz, on évalue les maïs présentant le meilleur rendement énergétique après transformation.

Afin de déterminer le caractère élite d'un maïs, on compare des hybrides obtenus à partir de celui-ci aux hybrides commerciaux de référence (vendus pour le même objectif dans la même région), par des essais en champs, par relevé et mesures de caractères agronomiques appropriés à l'objectif recherché. Un maïs est défini comme élite si les résultats obtenus paramètres étudiés pour un hybride obtenu par croisement dudit maïs sont supérieurs à 90 % aux résultats relevés pour les mêmes paramètres des hybrides de référence.

Ainsi, un maïs élite est un maïs rassemblant le maximum de caractéristiques agronomiques nécessaires pour une pénétration économique du marché visé. Le marché du maïs étant aujourd'hui un marché d'hybrides, l'évaluation du caractère élite d'un maïs s'effectue également par l'aptitude dudit maïs à la combinaison/production d'hybrides.

Ainsi, la mise en oeuvre de la présente invention permet d'obtenir un maïs élite destiné à la commercialisation d'hybrides pour toute utilisation, ledit maïs présentant une précocité de floraison. Ce maïs élite est donc homozygote pour l'allèle delta-Prec31.

Dans un autre mode de réalisation, l'invention permet d'obtenir un maïs hybride obtenu par croisement de deux lignées parentes homozygotes, ledit maïs hybride présentant un allèle delta-Prec31. Ce maïs hybride peut être homozygote (si chaque parent homozygote présente l'allèle delta-Prec31) ou hétérozygote pour l'allèle.

Toute plante telle que décrite plus haut (transgénique ou mutante) peut contenir un ou plusieurs transgènes en plus des éléments inhibant Prec31 et de l'allèle delta-Prec31. On peut citer des transgènes conférant une stérilité mâle, une fertilité mâle, la résistance à un herbicide (notamment le glyphosate, le glufosinate, l'imidazolinone, la sulfonylurée, la L-phosphinotricine, la triazine, le benzonitrile), la résistance aux insectes (notamment un transgène codant pour une toxine de *Bacillus thuringiensis*), la tolérance au stress hydrique. Ces plantes peuvent être obtenus en croisant lesdites plantes de l'invention avec d'autres plantes contenant lesdits transgènes. Alternativement, on peut co-transformer des plantes avec une cassette d'expression contenant plusieurs transgènes différents, dont ceux inhibant Prec31.

L'invention se rapporte en particulier à une méthode pour obtenir un maïs présentant une date de floraison précoce, comprenant l'introgression de l'allèle delta-Prec31 dans ledit maïs, comprenant les étapes consistant à :
a) croiser une première lignée de maïs présentant l'allèle delta-Prec31 avec un second maïs ne présentant pas ledit allèle,
b) effectuer un génotypage de la descendance obtenue et sélectionner les descendants présentant l'allèle delta-Prec31, et optionnellement ayant le meilleur génome ratio pour ce qui est dudit second maïs,
c) effectuer un rétrocroisement desdits descendants avec ladite seconde lignée de maïs élite utilisable pour la production d'hybrides,
d) répéter si nécessaire les étapes b) et c) jusqu'à obtenir une lignée isogénique dudit second maïs, présentant l'allèle delta-Prec31,
e) de façon optionnelle, effectuer une auto-fécondation afin d'obtenir une plante homozygote pour l'allèle delta-Prec31.

Lorsque la lignée de maïs présentant l'allèle delta-Prec31 est homozygote pour cet allèle, il n'est pas besoin de réaliser le génotypage prévu à l'étape b) après le premier croisement prévu à l'étape a).

Dans un mode de réalisation particulier, ledit allèle est représenté par SEQ ID N° 2.

Enfin, l'invention se rapporte à l'utilisation d'une plante, et notamment d'un maïs selon l'invention pour la préparation d'une composition destinée à l'alimentation humaine ou animale, ou à la préparation de biocarburants ou à toute autre application industrielle, ainsi qu'aux méthodes utilisant de telles plantes pour de telles applications.

### EXEMPLES

### Exemple 1 : caractérisation du mutant delta Prec31

Une lignée de maïs présentant une insertion d'un élément transposable après le G situé en position 6125 de la séquence de référence SEQ ID N° 2 est isolée. L'allèle ainsi obtenu est appelé delta-Prec31.

Bien que présente dans la région 3' non traduite du gène, on suppose que cette insertion a pour conséquence de déréguler la transcription et/ou la traduction du gène Prec31, ou la stabilité de l'ARNm de Prec31, menant à une diminution de l'activité de la protéine en présence de l'allèle delta-Prec31.

Afin d'étudier plus précisément l'effet de l'insertion observée dans le gène Prec31 dans un maïs élite, on effectue des back-cross (rétrocroisements) successifs avec une lignée élite de maïs.

Cette méthode permet très rapidement d'obtenir des lignées quasi isogéniques ne différant que par le locus portant l'allèle modifié, les descendants étant testés pour posséder un ratio génome le plus proche possible de celui du parent élite tout en ayant l'allèle que l'on désire introgresser. Ces tests sont assistés par marqueurs moléculaires (techniques bien connues, microsatellites, AFLP...). Pour essayer d'apprécier l'effet de l'insertion au plus tôt (obtention de plantes homozygotes), des autofécondations sont réalisées aux différents stades intermédiaires de back cross.

On évalue ainsi la précocité sur les plantes BC3S1 (3 rétrocroisements dans une lignée élite, 1 auto-fécondation).

On observe les résultats suivants
Floraison mâle (FM) : Précocification significative à l'état homozygote mutant par rapport à l'homozygote sauvage quasi-isogénique. L'effet est très net : P value = 0,0046 et différence significative de 5 jours entre les moyennes de floraison des homozygotes mutants comparées aux moyennes de floraison des homozygotes sauvages.
Floraison femelle (FF) : Précocification également significative à l'état homozygote mutant par rapport à l'homozygote sauvage quasi-isogénique. P value = 0,0221 et différence significative de 4 jours entre les moyennes de floraison des homozygotes mutants comparées aux moyennes de floraison des homozygotes sauvages.

La mutation engendre donc une précocification de l'ordre de 4 à 5 jours, tant pour la floraison mâle que pour la floraison femelle.

Afin de déterminer si l'insertion est sous forme homozygote ou hétérozygote, on peut utiliser un couple d'amorces :
SEQ ID N° 6 : GATTGCAGGACTCGATCAA, en amont de l'insertion, et une amorce de séquence SEQ ID N° 7 : TTTAACCCAAACACAACAGG, en aval de l'insertion.

En plus de ces deux amorces l'amorce OMuA SEQ ID N°8 : CTTCGTCCATAATGGCAATTATCTC spécifique de l'élément transposable endogène est utilisée. Cette amorce est dirigée vers « l'extérieur » du transposon. Ces trois amorces peuvent être utilisées simultanément dans une expérience d'amplification PCR à partir d'ADN génomique (Température d'hybridation = 58°C). Le dépôt sur gel des produits d'amplification révèle :
- l'obtention d'une seule bande d'environ 450 pb de long pour des plantes dites sauvages à ce locus (c'est à dire n'ayant pas la mutation) ;
- l'obtention d'une bande de l'ordre de 500 pb pour des plantes homozygotes mutantes
- ou l'obtention des deux bandes pour des plantes hétérozygotes.

### Exemple 2 : recherche d'autres mutations dans le gène Prec31

On utilise les amorces TGAGTGGACATGCTTGACC (SEQ ID N° 4) et CGTATAACACGGAAGCTGAC (SEQ ID N° 5) pour amplifier une région chevauchant la fin du second exon et le début du second intron du gène Prec31.

Ces amorces amplifient une région de 754 nucléotides allant du nucléotide 4803 au nucléotide 5556 de SEQ ID N°2.

On observe la présence de 3 polymorphismes, localisés à 4961, 5025 et 5089 de SEQ ID N° 2.

Ces nucléotides présentent l'effet suivant sur la date de floraison :

| Polymorphisme | Allèle1 | Effet | Allèle 2 | Effet |
|---|---|---|---|---|
| 4961 | C | 25,94 | A | - 25,94 |
| 5025 | T | 25,77 | G | - 25,77 |
| 5089 | - | 25,57 | T | - 25,57 |

Les effets sont indiqués en degré-jours. De fait, lorsque la variabilité interannuelle des températures est grande, on évalue la durée des cycles de développement, non pas en nombre de jours, mais en somme de degrés-jour. De façon simplifiée, la somme des degrés-jours correspond à la somme des températures journalières moyennes. Dans le cas présent, la température observée lors de la floraison étant de l'ordre de 20°C, les polymorphismes précocifient ou retardent la date de floraison d'un peu plus d'une journée.

La comparaison est effectuée par rapport à la moyenne des dates de floraison pour l'ensemble des lignées d'un panel de 374 lignées de maïs représentant une large diversité génétique. Les dates retenues pour chacun des polymorphismes correspondent à la moyenne des dates de floraison pour toutes les lignées contenant ledit polymorphisme.

Du fait de la grande variabilité entre les lignées utilisées, cette méthode permet de minimiser l'effet des autres loci génétiques pouvant être impliqués dans la floraison.

Le SNP le plus associé à la précocité est situé en position 4961 de la séquence de référence SEQ ID N° 2. Il est très significatif (associé avec une pvalue de 5.1 10⁻⁴) et présente un allèle précocifiant A de 25.94°jour et un allèle tardifiant C de 25.94°jour. Le pourcentage de variance phénotypique associé à ce locus est de 5%.

Il est à noter que la séquence de référence SEQ ID N° 2 fournie pour permettre de localiser les polymorphismes du gène Prec31 possède les polymorphismes de l'haplotype 2, et est donc associée à une floraison précoce.

Ces résultats confirment l'implication du gène Prec31 dans le mécanisme de floraison.

### Exemple 3 : expression de Prec31

Des échantillons tissulaires (apex, partie interne de la gaine et partie externe de la gaine) ont été récoltés à différents stades foliaire et notamment au stade 6,3 feuilles, c'est-à-dire le stade de transition florale (« switch » de la floraison) qui correspond à celui du passage du stade végétatif au stade floral.

On a pu montrer que Prec31 a une expression transcriptionnelle diffétrentielle entre la partie interne de gaine et la partie externe exposée à la lumière. Au stade 6,3 feuilles, le gène Prec31 est plus fortement exprimé dans la partie extérieure.

Ce profil d'expression est inverse celui du gène id1 (INDETERMINATE 1) chez le maïs (Colasanti et al, Cell, may 15,3(4) :593-603, 1998) connu pour induire la floraison.

Ces résultats, ajoutés au fait que le gène Prec31 colocalise avec un QTL de précocité de la floraison localisé sur le chromosome 8 (QTL au bin 8.05), confirment l'implication du gène Prec31 dans le mécanisme de floraison.

## Revendications

1. Procédé pour avancer la date de floraison d'une plante, **caractérisé en ce que** l'on inhibe totalement ou partiellement l'expression et/ou l'activité dans ladite plante, d'une protéine dénommée Prec31, dont la séquence polypeptidique possède au moins 75% d'identité avec la séquence SEQ ID N° 1.

2. Procédé selon la revendication 1, **caractérisé en ce que** ladite plante est le maïs.

3. Utilisation d'au moins un polynucléotide choisi parmi :
a) un polynucléotide codant pour une protéine Prec31 telle que définie dans la revendication 1 ;
b) un polynucléotide complémentaire d'un polynucléotide a) ci-dessus ;
c) un fragment d'au moins 12 nucléotides consécutifs, d'un polynucléotide a) ou b) ci-dessus, ou capable de s'hybrider sélectivement avec ledit polynucléotide ;
d) un polynucléotide permettant la production d'un duplex utilisable pour l'interférence d'ARN (RNAi), ledit un polynucléotide contenant un polynucléotide X contenant au moins 12 nucléotides, de préférence au moins 15, avantageusement au moins 20, et de manière tout à fait préférée au moins 50 nucléotides capable de s'hybrider sélectivement avec un polynucléotide défini en a), ainsi qu'un polynucléotide Y complémentaire dudit polynucléotide X
pour la mise en oeuvre d'un procédé selon l'une des revendications 1 ou 2.

4. Procédé selon une quelconque des revendications 1 ou 2, **caractérisé en ce que** l'inhibition de l'expression et/ou de l'activité de l'enzyme Prec31 est obtenue par mutagenèse du gène codant pour ladite enzyme [ledit gène étant entendu comme comprenant les régions codantes et non codantes (5', 3', introns) du gène].

5. Procédé selon une quelconque des revendications 1 ou 2, **caractérisé en ce qu'**il comprend la transformation de ladite plante avec une construction d'ADN recombinant comprenant un polynucléotide tel que défini dans la revendication 3, sous contrôle transcriptionnel d'un promoteur approprié.

6. Cassette d'expression comprenant un polynucléotide tel que défini dans la revendication 3, sous contrôle transcriptionnel d'un promoteur approprié.

7. Vecteur recombinant contenant une cassette d'expression selon la revendication 6.

8. Plante susceptible d'être obtenu par le procédé selon la revendication 4, et présentant une mutation dans le gène codant pour la protéine Prec31 dont la séquence possède au moins 75 % d'identité avec SEQ ID N° 1, ladite mutation résultant en une inhibition de l'expression et/ou l'activité de Prec31.

9. Plante génétiquement modifiée, susceptible d'être obtenue par un procédé selon la revendication 5.

10. Plante selon l'une des revendications 8 ou 9, **caractérisée en ce qu'**il s'agit d'un maïs.

11. Procédé de sélection de maïs, **caractérisé en ce qu'**il comprend la recherche d'un allèle du gène de la protéine Prec31 possédant une mutation résultant en une date de floraison anticipée dudit maïs par rapport à un maïs ne possédant pas cet allèle.

12. Procédé selon la revendication 10, caractérisé ce que ledit allèle est représenté par SEQ ID N° 2.

13. Maïs présentant un allèle du gène Prec31, appelé delta-Prec31 comprenant une insertion d'un transposon dans la région 3' non-traduite du gène Prec31, ladite insertion étant localisée après le G situé en position 6125 de la séquence de référence SEQ ID N° 2, ledit allèle étant présent dans un échantillon représentatif de graines déposées au NCIMB sous le numéro NCIMB 41706.

14. Grain de maïs présentant un allèle du gène Prec31, appelé delta-Prec31 comprenant une insertion d'un transposon dans la région 3' non-traduite du gène Prec31, ladite insertion étant localisée après le G situé en position 6125 de la séquence de référence SEQ ID N° 2, ledit allèle étant présent dans un échantillon représentatif de graines déposées au NCIMB sous le numéro NCIMB 41706.

15. Méthode pour obtenir un maïs présentant une date de floraison précoce, comprenant l'introgression de l'allèle delta-Prec31 dans ledit maïs, comprenant les étapes consistant à :
a) croiser une première lignée de maïs présentant l'allèle delta-Prec31 avec un second maïs ne présentant pas ledit allèle,
b) effectuer un génotypage de la descendance obtenue et sélectionner les descendants présentant l'allèle delta-Prec31, et optionnellement ayant le meilleur génome ratio pour ce qui est dudit second maïs,
c) effectuer un rétrocroisement desdits descendants avec ladite seconde lignée de maïs élite utilisable pour la production d'hybrides,
d) répéter si nécessaire les étapes b) et c) jusqu'à obtenir une lignée isogénique dudit second maïs, présentant l'allèle delta-Prec31,
e) de façon optionnelle, effectuer une auto-fécondation afin d'obtenir une plante homozygote pour l'allèle delta-Prec31.

16. Utilisation d'un maïs selon l'une des revendications 10 ou 13, ou obtenu selon la méthode de la revendication 15 pour la préparation d'une composition destinée à l'alimentation humaine ou animale, ou à la préparation de biocarburants ou à toute autre application industrielle.
